# EUROPEAN PATENT APPLICATION

(11) **EP 1 087 322 A2**
(43) Date of publication of application: **28.03.2001**
(21) Application number: 00120185.4
(22) Date of filing: 22.09.2000
(51) Int. Cl.: G06F 19/00

(54) **Method and apparatus for monitoring patient activity**

(30) Priority: 23.09.1999 US 155673 P; 19.06.2000 US 596587
(71) Applicant: RXVP, Inc., Chester, Pennsylvania 19013 (US)
(72) Inventor: Nalbone, Samuel A., Jr., Wayne, Pennsylvania 19087 (US); Berlet, Jeffrey L., Collegeville, Pennsylvania 19426 (US); Martin, Roger I., State College, Pennsylvania 16804 (US)
(74) Representative: Schwabe - Sandmair - Marx

(57) **Abstract**

Patient compliance with a medication regimen is tracked. Information regarding time and dosage requirements of medication for a patient is maintained. The patient is signaled based on the information, a pathway is provided to receive from the patient indications of compliance responsive to the signaling and a third party is provided with an indication of whether the patient has complied with the requirements based on whether the indications have been received.

## Description

### FIELD OF THE INVENTION

The present invention relates to a communication method and apparatus and, more specifically, to a method and apparatus for reminding patients to follow a medication regimen. In particular, a method and system are disclosed that transmits medication reminders to patients and provides for third party monitoring of compliance.

### BACKGROUND OF THE INVENTION

It is a harsh reality that healthcare costs are rising to historically high levels. One of the methods that healthcare providers can use to reduce the cost of patient care is to reduce the cost of delivering the care to the patient. One way to accomplish this is to move patients out of hospital and hospital-type care as soon as possible and to care for patients at home.

Many home-care patients require monitoring. Traditionally, extensive human involvement has been necessary, thus keeping the costs of monitoring high. Unfortunately, the high costs of patient monitoring usually cause only the most extreme case to receive the high level of care associated with in-home monitoring. Meanwhile, a large segment of patients do not receive the monitoring that they need. This is especially true in the use of drug therapy.

Another trend in modem healthcare is the extensive use of pharmaceutical products. A patient may be on a medication regimen involving multiple drugs, which need to be taken at varying frequency and dosage. As such, a patient may have problems remembering when to take which medication or if a medication has been taken.

The relationship of medication compliance to overall patient well being is poorly documented but generally considered as a critical variable in predicting patient health. The problem for many outpatients is taking medication as prescribed. Prescriptions involve dosage as well as proper timing to optimize the effectiveness of medication while reducing harmful or even unpleasant side effects. Forgetting to take medication is a very frequent and unfortunate situation. Many patients report not knowing if they have taken medication and "play it safe" by taking medication again. Concurrently, there are issues for care givers, family members, insurers, and pharmaceutical related firms that wish to improve their understanding of patient compliance and the therapeutic value of medications under varying compliance levels.

A current trend to solve these issues is to use communication technology to monitor the patient. A currently popular and low cost method of communication is the wireless pager. Pagers are small, relatively inexpensive, easily transported, and easy to use. A patient wearing a pager can be contacted wherever the patient may be. The patient need not be in a particular room of his or her home, or even at home at all, to be contacted. Pager technology has advanced to a point where messages can be broadcast to a small display embedded in the pager. Also, bi-directional communication is possible. The patient may respond to a message sent to the pager with a simple push of a button.

The Internet has also grown in popularity and ease of use. The Internet can provide almost instantaneous worldwide communications. Although it requires the use of a computer, most businesses, including health care providers, have computerized their record keeping functions and billing functions. Pharmacies keep patient records in computer databases. Even if these organizations were not yet using the Internet, it would be simple to supply access. Individuals who do not have Internet access already can connect through inexpensive hardware such as WebTV®. Internet service providers are also lowering the cost of hardware, and, in some cases, giving away the computer in order to sell Internet access.

Still, large portions of the population have limited exposure to technology and advanced electronic devices such as digital phones, pagers, computers, and even advanced functions on VCR's and televisions. Other individuals with technology experience and interest also want simplified devices to replace currently complicated tools. "Smart" and dynamic devices that are simplified can be put to use in solving medical compliance and patient management problems. The device capabilities of phones, pagers, televisions and other tools surpass individual patient needs and interest and the resulting "clutter" of information precludes adoption of the technology by more people than currently own advanced cell phones and pagers. Complexities are introduced through the enabling software, and the software/hardware interface structure.

### SUMMARY OF THE INVENTION

The present invention relates to a method and apparatus for tracking patient compliance with a medication regimen. Information regarding time and dosage requirements of medication are maintained for a patient. The patient is signaled based on the information. Also provided is a pathway to receive from the patient indications of compliance responsive to the signaling. A third party may receive an indication of whether the patient has complied with the requirements based on whether the indications have been received.

### BRIEF DESCRIPTION OF THE DRAWINGS

Figure 1 is a block diagram of an exemplary implementation of the present invention.
Figure 2 is a block diagram that illustrates an overview of an exemplary embodiment of the present invention.
Figure 3 is a block diagram that illustrates an overview of an exemplary embodiment of the present invention
Figure 4 is an illustration of an exemplary configuration of the Logbook database of the present invention.
Figure 5 is an illustration of an exemplary configuration of the RXVP database of the present invention.
Figure 6 is an illustration of an exemplary configuration of a medication table of the present invention.
Figure 7 is an illustration of an exemplary configuration of a Prescription Event Table of the present invention.
Figure 8 is an illustration of an exemplary configuration of a Compliance Table of the present invention.
Figure 9 is an illustration of an exemplary configuration of a Reminder Table of the present invention.
Figure 10 is a block diagram that illustrates the relationship between the various components in accordance with the exemplary embodiment.
Figure 11 is a flow chart that illustrates the operation of the messaging routine of an exemplary embodiment of the present invention.
Figure 12 is a flow chart that illustrates the operation of the compliance routine of an exemplary embodiment of the present invention.
Figure 13 is a flow chart that illustrates the operation of the observer routine of an exemplary embodiment of the present invention.
Figure 14 is a block diagram that more clearly illustrates the operation of the RXVPBean shown in Figure 10.
Figure 15 is a block diagram that more clearly illustrates the operation of the TerminalBean shown in Figure 10.
Figure 16 is a block diagram that more clearly illustrates the operation of the ComplianceBean shown in Figure 10.
Figure 17 is a block diagram that more clearly illustrates the operation AdministrationBean shown in Figure 10.
Figure 18 is a block diagram that more clearly illustrates the operation of the BillingBean shown in Figure 10.
Figure 19 is a block diagram that more clearly illustrates the operation of the AnalysisBean shown in Figure 10.

### DETAILED DESCRIPTION OF THE DRAWINGS

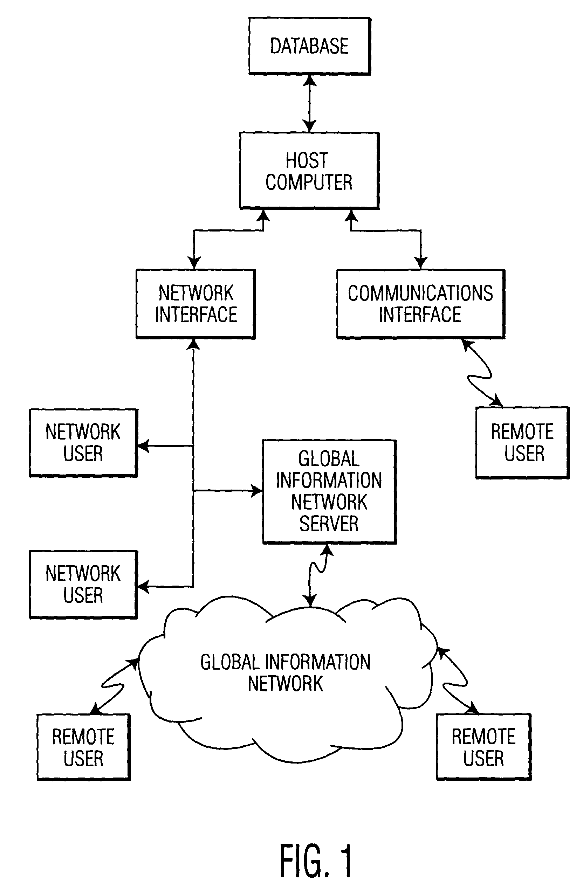

The method of the present invention is, in an exemplary embodiment, executed using a general-purpose computer network and software as shown in Figure 1. While the following description assumes this exemplary configuration for execution, it is understood that other computerized systems may also be used.

In an exemplary embodiment of the present invention, a system (hereafter "the RXVP system") sends a communication signal to a patient. The purpose of the communication signal may be, for example, to remind the patient to take his medication. The signal can be transmitted in a variety of ways. The transmission can be an e-mail, a signal sent to a pager device, or a telephone call. An acknowledgement signal is then transmitted back to the RXVP system (typically) in the same format that it was transmitted to the patient. It is understood, however, that the acknowledgement signal need not be transmitted back to the RXVP system in the same format (or using the same medium) that was used to transmit the initial signal to the patient. For example, the patient may receive a text message which the patient may respond to by using a telephony signal. Thus, the patient may, for example, receive a text message by e-mail (or pager) and then respond to the message using a telephone. Alternatively, for example, the patient may receive a reminder by telephone and may then provide a response to the RXVP system using a form of data transmission (e.g. e-mail). Thus, the patient reminder and the patient response, although occurring over multiple (and respectively different) communication mediums, are desirably generated and maintained from a common location. This facilitates maintaining relationships between the patient reminders and the anticipated acknowledgements. A third party observer can then immediately be notified that the acknowledgement signal from a patient has been received by the RXVP system. This notification signal can be transmitted to the third party observer in any of the ways mentioned above, however, it need not be the exact same type signal as the communication signal sent to the patient. Or, if desired, the third party observer can access the RXVP system through the Internet ( or other network) to view a report of any signals received from the patient. For example, the system may send an e-mail to a patient and the patient may respond with an e-mail. Immediately, a phone call can be placed to that patient's third party observer to inform the third party observer that the patient is complying. Or, if desired, the third party observer can access the system through the Internet at a later time, to see if the patient has complied with his medication regimen. The system can mix different signaling and communication mechanisms, so that, if a patient is more comfortable using a pager, but a third party observer is more comfortable using the Internet, both users of the system can use their preferred communication method to obtain the desired results.

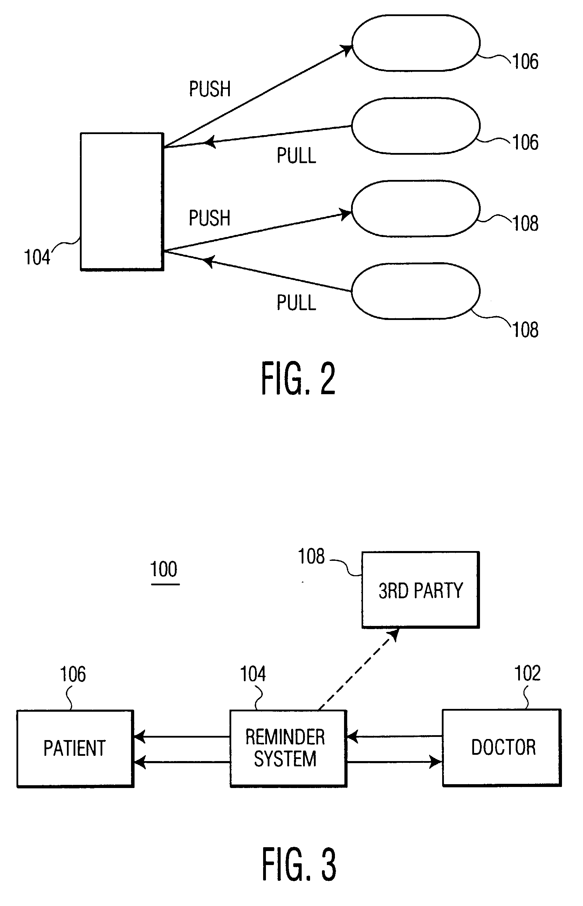

An exemplary embodiment of the system overview described above is illustrated in Figure 2. The patient reminder system 104 can push (i.e., transmit without a request from the recipient) a message out to a patient 106 or a third party observer 108. This can be accomplished by the sending of an e-mail, a telephone call, a signal to a paging device, or other communication means. Also, a patient 106 or a third party observer 108 can pull (i.e., cause the transmission based on a request from the recipient) the information from the patient reminder system 104 by accessing the system through, for example, the Internet or an Intranet. The push signal from the patient reminder system 104 to the patient 106 need not be the same as the pull signal used by patient 106 to access the patient reminder system 104. Likewise, the push signal to the third party observer 108 need not be the same type of signal as the pull signal from observer 108 to the patient reminder system 104.

Another overview of the present invention is illustrated, for example, through the block diagram which is shown in Figure 3. As shown in Figure 3, operation of the present invention begins with Doctor 102. Doctor 102 will prescribe a prescription for a patient. The prescription includes several components including the type of medication, the quantity of medication, the timing with which the medication is to be taken, the concentration of the medication, etc. Doctor 102 conveys this prescription information to patient or reminder system 104. Patient or reminder system 104 keeps track of prescription information for a variety of patients. At the appropriate time, for patients to be reminded to take their medication, reminder system 104 conveys an appropriate reminder signal to patient 106. Patient 106 receives the reminder signal from patient reminder system 104. After patient 106 takes the medication, patient 106 then provides a return signal to the patient reminder system 104. The return signal indicates that patient 106 has indeed taken the medication as per the doctor's prescription. Patient reminder system 104 keeps track of the signals which patient 106 provides in response to the reminder signal which is initiated from patient reminder system 104. Doctor 102 then has access to the various records maintained by patient reminder system 104. These records may include, for example, the level of compliance that patient 106 is maintaining with regard to the doctor ordered prescription. An optional third party 108, also has access to this compliance information.

In order to better understand the operation of patient reminder system 104, several data structures are used with which to organize and track the various data. Before describing the various processes which are used to organize and track the data, it is helpful to explain the data structures which are used by patient reminder system 104. Those data structures which are used are the following:
1) Logbook Table,
2) RXVP Database,
3) Medication Table,
4) Prescription Event Table,
5) Compliance Table, and
6) Reminder Table.
Each of the above data structures will be discussed separately.

### 1) Logbook Table:

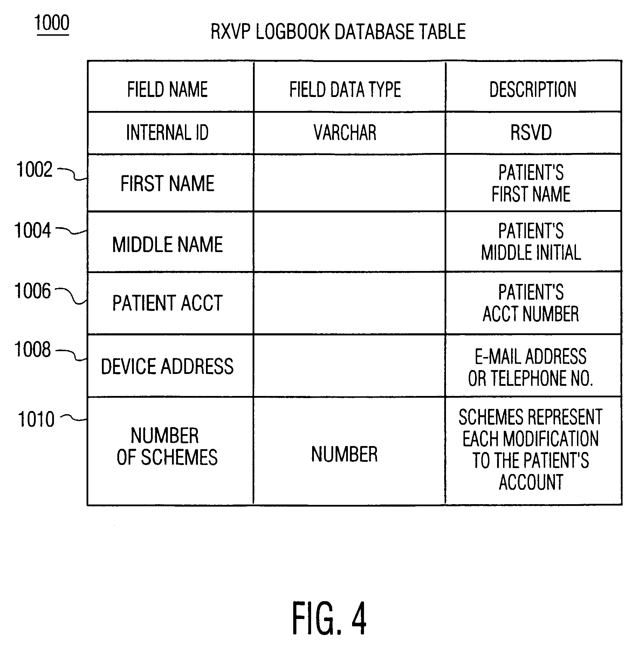

An exemplary embodiment of the logbook table is shown, for example, in Figure 4. The logbook table shown in Figure 4 is merely exemplary. As shown, the logbook table includes three separate columns including field name, field data type, and description. The purpose of the field name is to indicate the name of the data field in the database. As shown in this exemplary embodiment, there may be, for example, six fields. An internal ID field is used for internal tracking, A first name field 1002 identifies the first name of the patient. A middle initial field 1004 identifies the middle initial of the patient. A patient account field 1006 identifies an account number for the patient. A device address field 1008 identifies a device address for the patient. The device address may be, for example, a particular email address, a particular telephone address, a particular pager address, etc. Number of schemes field 1010 indicates the total amount of medications per device. For example, if a patient is taking 4 medications and is receiving reminders over 2 devices, this field may include, for example, the number 8.

### 2) RXVP Database:

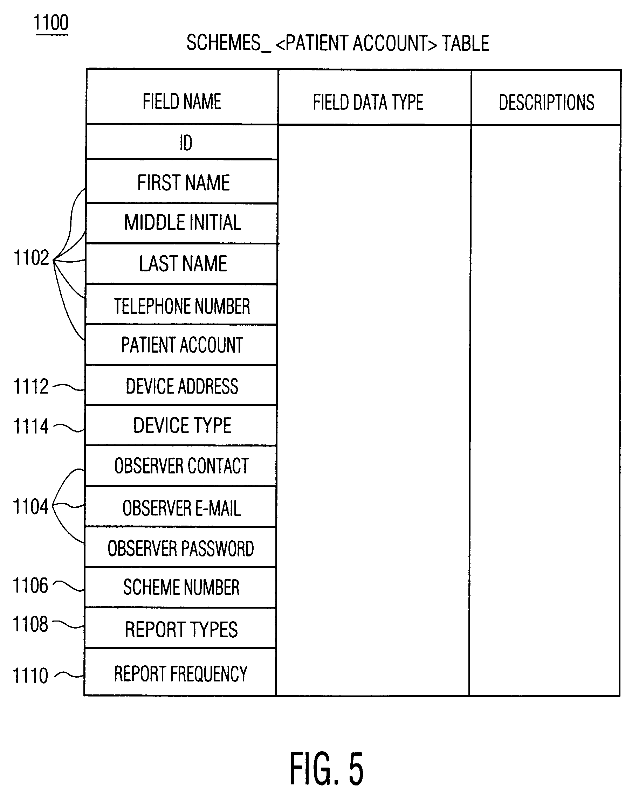

An exemplary embodiment of the RXVP database is shown, for example, in Figure 5. For reason of identification, the RXVP database is identified as RXVP database 1100. As shown in this exemplary embodiment, RXVP database 1100 includes 3 columns, namely, field name, field data type, and description. Each field will be described separately. Fields 1102 identify the patient. Thus, fields 1102 include personal information such as name, phone number, patient account number, etc. Device address field 1112 indicates the address of the device to which reminders are to be transmitted. As indicated above, for example, the device address may correspond to a telephone number, a pager, an email address, etc. Device type field 1114 indicates the actual device type, e.g. phone, pager, etc. Fields 1104 are used to identify a third party observer if, a third party observer is used. Thus, fields 1104 include information such as an observer password, an observer email address, etc. Field 1106 is a scheme number which identifies the total number of types of reminders for a patient. Thus, for example, if there are 4 types of medications and 2 devices by which reminders are to be sent, there will be, for example, a total of 8 separate schemes. Finally, fields 1108 ad 1100 indicate the types of reporting and the frequency of reporting, respectively.

### 3) Medication Table:

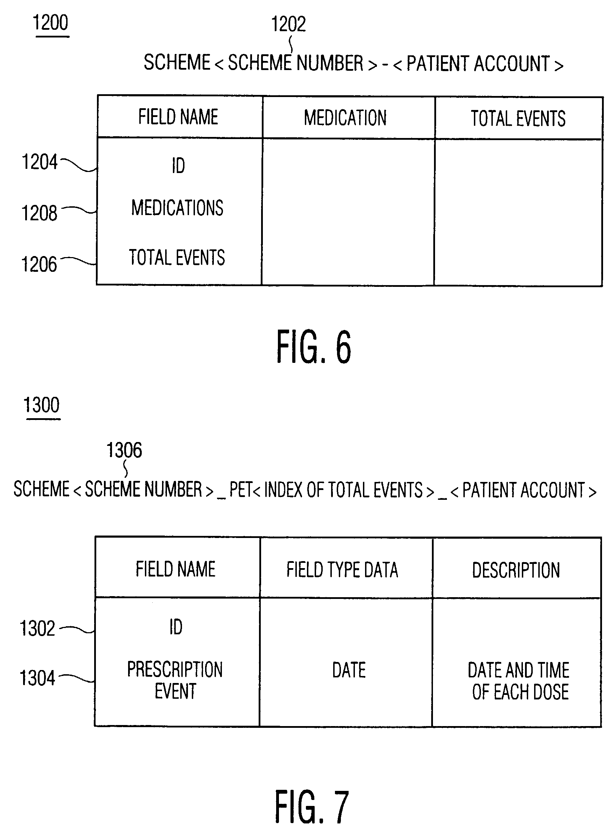

Figure 6 is an exemplary embodiment of a medication table. The medication table contains 3 columns. The first column, field name, describes the fields contained in the medication table. The identification field 1204 is used to hold a unique identifier for patient. The medication field 1208 contains a description of the medication or medications taken by the patient. The total events field 1206 contains the total number of messages that are to be sent per day to the patient to which this table refers. The title of the table contains a scheme number 1202 which is used to index this table to the other tables referencing the medications in the medication table 1200.

### 4) Prescription Event Table:

Figure 7 is an exemplary embodiment of the prescription event table 1300. A prescription event table contains 3 columns, namely, field name, field data type and prescription. The identification field 1302 contains the unique patient identification number. The prescription event field 1304 contains the date and time for each message to be sent to remind the patient to take their medication. The title of the table contains scheme number 1306 which is used to index and match this prescription event table to the proper medication table.

### 5) Compliance Table:

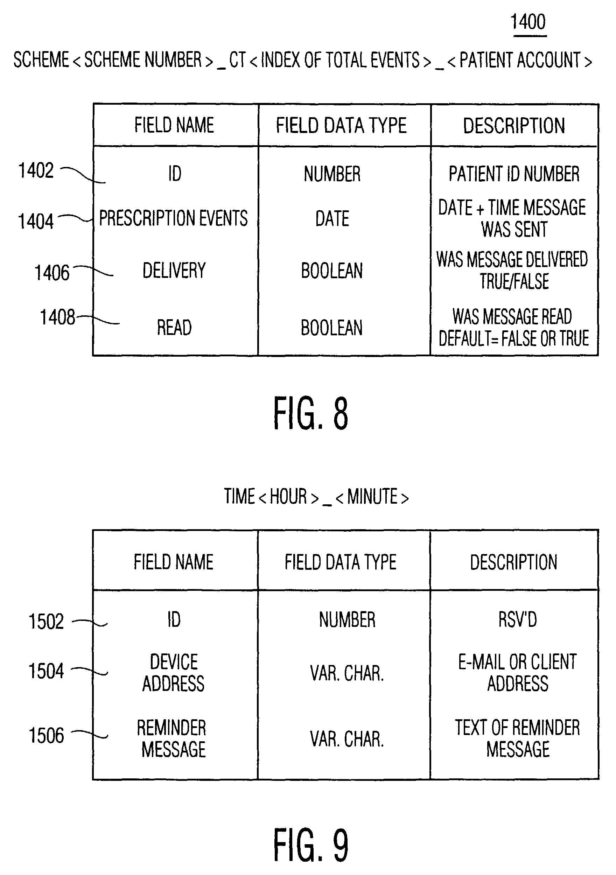

Figure 8 is an exemplary embodiment of the compliance table 1400. The compliance table contains 3 columns, namely, field name, field data type and description period. The identification field 1402 is used to hold the unique patient identification number. The prescription event field 1404 holds the date and time at which the message is sent to the patient. The delivery field 1406 holds a Boolean true/false which indicates if the message was delivered. The read field 1408 is a Boolean true/false field. The read field 1408 is set to a default of false. If a compliance signal is received, this field is set to true.

### 6) Reminder Table:

Figure 9 is an exemplary embodiment of the reminder table 1500. This table contains 3 columns, namely, field name, field data type and description. The identification field 1502 is reserved. The device address field 1504 holds the address of the device to which messages are to be sent. This could be an email address, a telephone number, or a beeper address. The reminder message field 1506 contains the text of the reminder message to be sent to the patient.

Having described the various data structures which may be used in an exemplary embodiment of the present invention, it is now useful to describe the various processes which interact with these data structures in order to cause exemplary embodiments of the invention to operate in the intended manner. Accordingly, various processes which may be used in accordance with an exemplary embodiment of the present invention are described below.

Before doing so, however, it is helpful to provide a general overview of operational flow of the exemplary components which may be used in implementing an exemplary embodiment of the present invention.

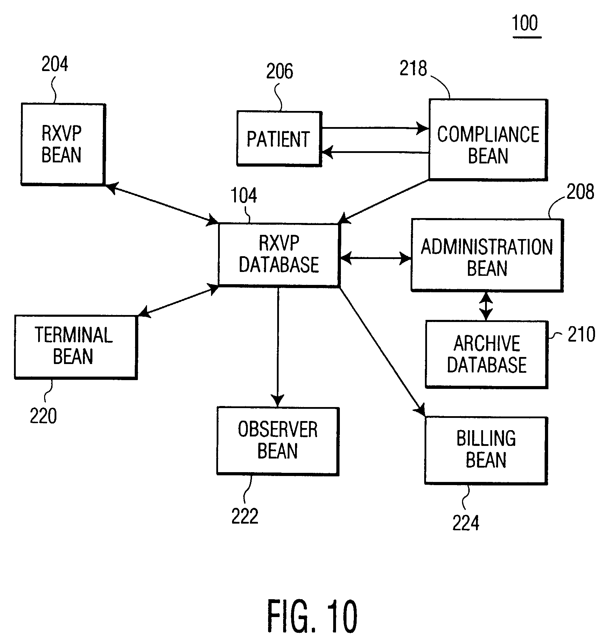

An exemplary embodiment of the various processes which communicate an exemplary embodiment of the present invention is shown, for example, with reference to Figure 10. Each of the items in Figure 10 will first be summarized.

### 1) RXVP Bean 204:

The purpose of RXVP Bean 204 is to send messages to a patient based upon the information which resides in RXVP database, reminder table, and prescription event table.

### 2) Patient 206:

Patient 206 is the actual patient which receives messages from RXVP Bean 204.

### 3) Compliance Bean 218:

The function of the Compliance Bean 218 is to receive the compliance messages from the patient, update the compliance table, and send a compliance signal back to the patient indicating that the patient's compliance information has been received.

### 4) Administration Bean 208:

The purpose of Administration Bean 208 is to create reminder tables, and to archive all data.

### 5) Archive Database 210:

The purpose of Archive Database 210 is to archive data which is old, i.e. that data which has not be accessed for a predetermined (often lengthy) amount of time.

### 6) Billing Bean 224:

The purpose of Billing Bean 224 is to gather information so that invoices may be generated for use of the present system.

### 7) Observer Bean 222:

The purpose of Observer Bean 222 is to provide an interface for a third party observer to review compliance reports.

### 8) Terminal Bean 220:

The purpose of Terminal Bean 220 is to provide the graphical interface for all remote users of the system.

### 9) RXVP Database 184:

RXVP Database 184 is the database in which the database structures described above with references to Figures 5-9 is stored.

Having briefly described each of the processes which cooperate in exemplary embodiment of the present invention, a more detailed explanation of these processes is now provided.

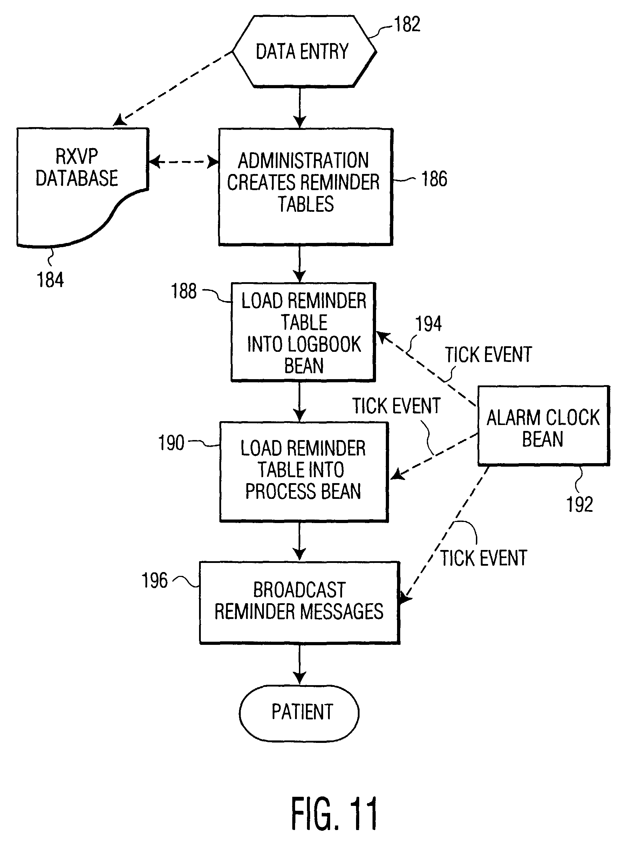

Turning to Figure 11, there is shown a block diagram of an exemplary embodiment of the patient messaging portion of present invention 180. In step 182, data is entered into the system. This data may be input locally, or may be input from a remote site via a LAN, WAN or the Internet. A medical provider, patient or pharmacist enters the proper information regarding the patient, including, at a minimum, the type of medication, the timing and the amount of the dosage, and personal identification data such as name, address and social security number. Other information may be entered as well, such as medical history, third party emergency contacts, insurance information and billing information. The data entered in step 182 is saved in the RXVP database 184. The RXVP database is preferably at least one relational database, and may be multiple relational databases. An exemplary configuration of the database is illustrated in Figure 5. As the data is entered, it is matched with data already existing in the database for the same patient, if any.

At step 186, the AdministrationBean 208 updates reminder tables. At system initialization, the tables are created from the database. At any other time, the tables can be updated with manual input and through use of the AdministrationBean 208. A reminder table is a subset of the central database and includes a device address and a message. The tables are built using common SQL programming language commands, which are generated by the AdministrationBean 208. The tables are indexed to the time of day at which the messages in the table will be broadcast to the appropriate patient. If messages are sent every 15 minutes, 96 (24 x 4) reminder tables are created for each day. By creating reminder tables indexed to the time of day, the complete database need not be searched each time a message needs to be broadcast, thus increasing the efficiency of the system. A complete set of reminder tables required for one day is created at system initialization. The reminder tables can be updated at any time via the AdministrationBean 208. An exemplary configuration of a reminder table is illustrated in Figure 9.

At initialization and after the reminder tables are created, the first reminder table to be broadcast is loaded in the Logbookbean at step 188. At step 190, the Logbookbean sends a ProcessPatient Event to the Processbean for each unique message in the reminder table loaded in the Logbookbean. The ProcessPatient Event contains a device address and the message. The next reminder table to be broadcast is then loaded into the Logbookbean repeating step 188. Also, the Alarmclockbean 192 is synchronized to the time-of-day clock of the system.

In this exemplary embodiment, a message interval of 15 minutes has been predetermined. Therefore, every 15 minutes, at step 194, the Alarmclockbean sends a Tickevent, or a pulse, to the Logbookbean, the Processbean and the Mailbean. When the Processbean receives a Tickevent, at step 196, the Processbean sends the reminder table loaded in the Processbean to the Mailbean for broadcasting to the patient. Repeating step 190, the Logbookbean then sends the reminder table loaded in the Logbookbean to the Processbean. Repeating step 188, the Logbookbean then retrieves the next reminder table in the sequence. At step 172, the Mailbean broadcasts the message to the patient. The system then waits for the next Tickevent before sending a new set of messages.

A message can be broadcast to the patient via a radio-frequency signal, telephone or via a computer network, and received by a commonly known beeper, telephone, or via a worldwide communications network such as the Internet, to inform the patient that medication must be taken. The ID of the messaging device is stored in the database. For messages broadcast at different times of day, different messaging devices can be addressed, so that, for instance, a message broadcast in the morning can be sent to a beeper, and a message broadcast in the evening can be sent to a Internet terminal. This feature of the system empowers the user to determine the best way to communicate and/or interact with the system, making communication with the system more effective.

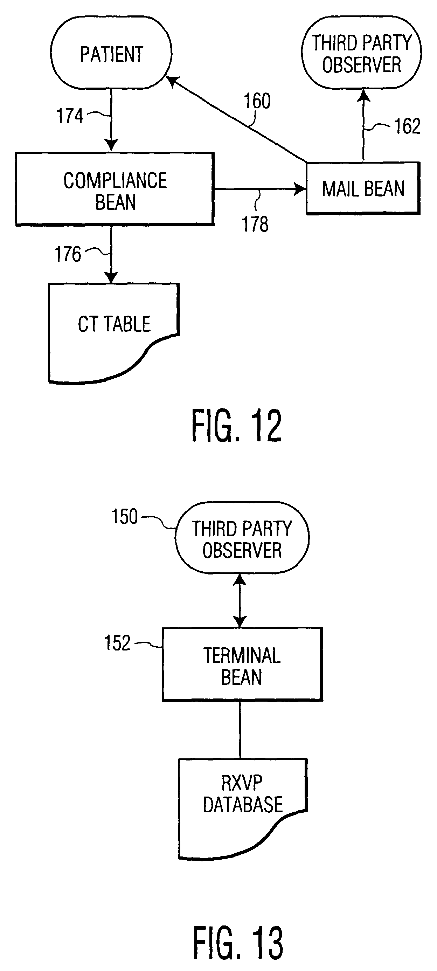

As illustrated in Figure 12, while the messaging routine of the present invention is functioning, the system is also receiving compliance indicators from the patients who received reminder messages. At step 174, the patient, by pressing a button on a beeper device, or replying to an e-mail message or a telephone call, can send a compliance indicator to the RXVP computer. The compliance signal is received by the ComplianceBean, which checks for the ID of the messaging device and the time the message was sent. At step 176. the ComplianceBean adds a record to the appropriate compliance table, and compares the time the message was received for each device address with the time the last message was sent. If the time differential is within a predetermined parameter, which, in this exemplary embodiment is 20 minutes, the "read" field in the compliance table is updated to indicate the compliance. Also, at step 178 the ComplianceBean will send a message to the Mailbean, for broadcasting a compliance signal to a third party observer at step 162, if appropriate, indicating that a compliance signal was received or not received. Also, at step 160, the Mailbean can send a confirming message to the patient.

As illustrated in Figure 13, while the messaging and compliance routines are operating, third party observers 150 may access the system to obtain compliance information on a specific patient. The system may be accessed through the TerminalBean 152 via the Intranet, or any communication network that can connect with the central server. A third-party observer can either receive reports on a pre-determined schedule, such as whenever the patient is non-compliant, or once per day. Also, the observer can access the real-time compliance data via the Internet at anytime on any browser.

In order to reduce CPU overhead at the server, when an observer 150 requests data, unformatted data is downloaded to the observer through the TerminalBean 152, along with a data-formatting program that will run on the observer's system. This feature also allows the user to reformat the data locally to produce an enhanced report without reestablishing or continuing the communication link with the RXVP system 104.

Figure 10 is an overview of the various components that comprise an exemplary embodiment of the present invention. In this exemplary embodiment, these components can be implemented in Java programming language. The software may be run on a general-purpose computer functioning as a server in a worldwide computer network such as the Internet. The RXVP database 184 is at the center of the system. The database 184 contains all the patient data, medication data and other data required for operation of the invention. In this exemplary embodiment, the database 184 resides in a single general-purpose computer that functions as a server in general purpose computer network. The database 202 may be a single or multiple databases. The RXVPBean 204 loads reminder events from each reminder table. At a tick-event generated by the Alarmclockbean (not shown), the RXVPBean 204 sends the reminder to the patient 206 via radio-frequency, telephone or digital communications such as a local area network, wide area network or a world-wide network such as the Internet. The RXVPBean 204 then automatically reloads the next scheduled reminder. If the system fails, the RXVPBean 204 resets, reloads, sends interrupted reminders and logs the failure.

The ComplianceBean 218 collects the compliance responses from the patients 206 and updates the compliance tables. An exemplary compliance table is illustrated in Figure 8. If a reminder is broadcast and the compliance response returned within a certain predetermined time, the appropriate field in the compliance table is updated. The default is no-compliance. The ComplianceBean 218 sends a message to an observer to inform the observer if a compliance signal was received.

The AdministrationBean 208 creates the pre-processed reminder tables that are used to generate messages at the appropriate times. The tables are a subset of the RXVP database and are created so that the entire database need not be searched for each Tickevent. Reminder table update can be accomplished via a user inputted update signal or automatically at preset times. Also, the AdministrationBean 208 stores, updates and retrieves retired patient records to and from the Archive database 210. By archiving records that no longer active, the database is more manageable and the time required for searching, sorting and other database applications is reduced.

The TerminalBean 220 is a local Intranet interface to the system. Medical providers can enter medical, administrative, and other data into the RXVP database 202 from a remote location. Also, patients and third party authorized observers can retrieve compliance data of a particular patient. Authorized personnel can also obtain statistical information regarding the medications used, compliance with the medication regimen and results of the treatment. A remote user can communicate with the database 184 via any well known communications software and hardware combination, including, but not limited to, local area network, wide area network, Internet, and the like.

The ObserverBean 222 allows authorized personnel to monitor the compliance of the patients 206. The database 184 may be accessed from a remote site using the functionality of the ComplianceDisplaybean. The observer must perform an activity consistent with the database's security features to access the database. This may be entering a simple password, or complying with more complex user identification protocols. Once the database is accessed, the observer is limited to viewing authorized data only. The data can be manipulated by the observer and displayed and/or printed in a variety of formats such as pie charts, line charts, line graphs, bar graphs, as the like. The raw data is downloaded to the observer along with a data-formatting program, so that the graphical processing occurs at the observer's computer system, at not at the server. This allows the recipient to reformat the data into a custom chart without having to predefine the report parameters with the TerminalBean 220.

The BillingBean 224 automatically compiles the data necessary for generating invoices. An interface is provided that links to conventional business software for producing invoices for regular and value-added services.

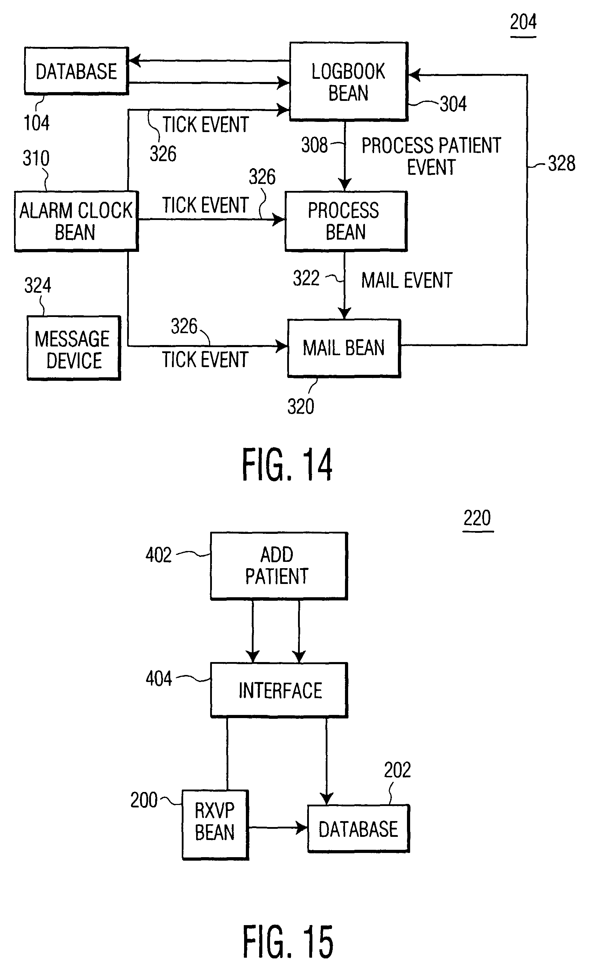

Figure 14 is a block diagram of the RXVPBean 204 component of the present invention. The RXVPBean 204 performs the task of sending the prescription events at prescribed intervals on a daily basis. Upon initialization, failure recovery, interval reset, or a Tickevent, the Logbookbean 304 loads the prescription events from the reminder tables already processed by the AdministrationBean (not shown). Any table already loaded in the Logbookbean is sent to the Mailbean 320 for broadcasting to the patient. The Logbookbean 304 checks to make sure that the time index for the reminder table matches the time clock for the next event. For each unique message in the current reminder table, a ProcessPatientEvent 308 is sent to the Processbean 306 with the appropriate device addresses and message. If there is more than one message, a set of device addresses per message is sent for each message. In this manner, the Processbean 306 can send one communication to a collection of patients instead of one communication for each patient. The Alarmclockbean 310 times the messages loaded into the RXVPProcessBean 306. When the message is due to be sent, it is passed to the Mailbean 320 through a MailEvent 322. The Mailbean 320 opens the communication port and forwards the message to the patient's messaging device 324. Delivery of the message and compliance data is sent to the Logbookbean 304 for entry into the database 184. Once a message is sent, the Logbookbean 304 replaces the sent message with the next message in the reminder table in the database 184 and the Logbookbean 304 loads the next reminder table.

The Alarmclockbean 310 produces a Tickevent 326 on a regular pulse with a predetermined period. The pulses are set at start-up so that the prescribed period is an integer divisor of 60 minutes. In an exemplary embodiment of the present invention, this period is 15 minutes. In this manner, there will be 96 pulses per day, which correspond to 96 reminder tables. These are the only times necessary to send prescription events. Prescription events are loaded from the preprocessed reminder table and composed by the Logbookbean 304 that then further groups the device addresses having identical messages. For each unique message there is an array of address objects. The address objects are then passed to the Processbean 306. At the proper pulse, the Processbean 306 send the messages with the address arrays to the Mailbean 320 to send the messages to the proper patients.

Figure 15 is a block diagram of the TerminalBean 220. The RXVP TerminalBean 220 comprises the AddPatientBean 402 and an interface 404 to the database 184. The interface allows SQL commands to be sent to the database 103 for database operations. The TerminalBean 220 provides an interface for third party personnel to access the RXVPdatabase 184. This may be performed directly, or through the AdministrationBean 208.

In an exemplary embodiment of the present invention, medical personnel will have a patient messaging device in their clinic or office to demonstrate and test the system for a patient prior to patient use. The medical personnel may enter data and test the system on a real time basis, as the database is immediately updated and events are immediately processed once the data is entered.

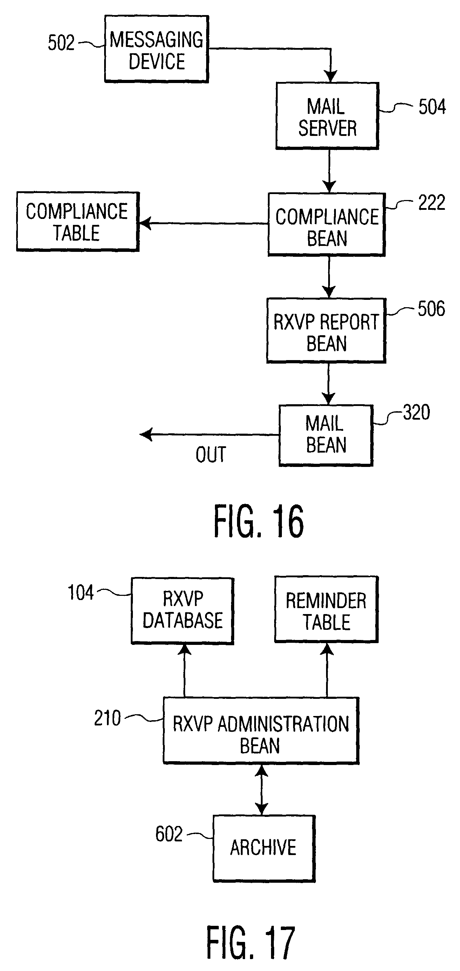

Figure 16 is a block diagram of the ComplianceBean 222. This component receives compliance data from a patient-messaging device 502 such as a radio-frequency device, a digital device or a computer network and sends that data to the database 184 to update the compliance table for a specific patient. The compliance data is immediately available for observation by authorized personnel. The ComplianceBean 222 receives messages from patients in a similar manner that e-mail users received e-mails. The messages are queued by the mail server 504, which is able to identify the individual patients even if the message was originally sent as a single communication to a plurality of patients. Therefore, even though the outbound communication may have been grouped into sets, the return messages arrive asynchronously.

In this exemplary embodiment, the ComplianceBean 222 reads the device address and time of message only. It is assumed that a message is a compliance message, as any reply is treated as a successful reminder response, so the actual message does not need to be read. The system also supports scanning of the reply message for keywords or numbers which indicate a specific response to a particular communication. For example, if the reminder message is for a patient to check his or her blood pressure, the response message may contain the blood pressure as a numeric value which can be read from within the response message and logged in the RXVP database 104.

The ComplianceBean 222 then compares the time of the message with the time of the prescription events in the database corresponding to the patient's device address. If the time differential falls below a predetermined upper limit, for example, 20 minutes, the message is determined to be a compliance message and the proper field in the compliance table is updated. At that point, if the optional report function is enabled, the ReporterBean 504 builds a compliance report and sends the report out through the Mailbean 320 to the proper third party observer. If the time differential is greater than the predetermined threshold, or if no acknowledgment is received, the optional report function is enabled, the ReporterBean 504 sends a non-compliance report out through the Mailbean 320. Additionally, the report function allows for communication of compliance and non-compliance reporting per event, or in a summary fashion for a predetermined set of events.

Figure 17 is a block diagram of the AdministrationBean 208. This component transfers inactive entries from the RXVP database 184 to archive databases 602 for record keeping and analysis. The functions performed by this component are account look-up and field editing, cancel account services and reset compliance data. This component will automatically update the reminder tables at a predetermined interval, chosen by the user. This component can be manually run as well.

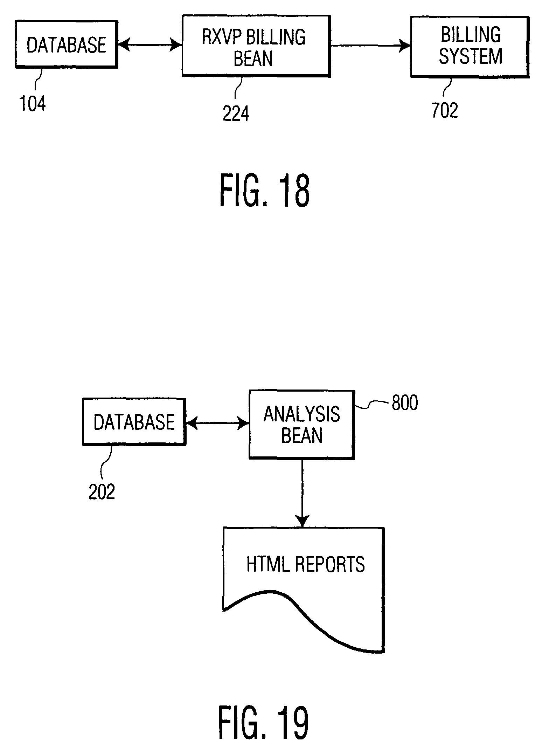

Figure 18 is a block diagram of the BillingBean 224. This component interfaces with the RXVP database 184 and compiles monthly data according to activation and termination dates of messaging service for each patient. The data is then passed to a billing system 702 for invoice generation and the like.

Figure 19 is a block diagram of the AnalysisBean 800. Statistical analyses specific to each medication are built into this component. The duration of the symptoms and length of prescriptions are used in the analysis. The effect of compliance on the patient's health and the reduction of side effects are evaluated. Results are compiled monthly and forwarded to authorized medical personnel.

While the exemplary embodiments have been described with regard to a patient being reminded to take his medicine, it is understood that the present invention may relate to more generalized types of reminder scenarios. For example, it is understood that exemplary embodiments of the present invention may be used to perform other types of reminder functions such as relating to errand type reminders (i.e. for an individual to adhere to any type of schedule) or to a diet reminder (i.e. to remind an individual to adhere to a food regimen), as well as any other type of scenario where it is desirable to remind an individual to perform a certain act. In this case, time and event requirements of one or more actions to be taken by one or more individuals are maintained. Time requirements are the times at which individuals are to perform the respective events and event requirements are descriptions (or other indicators) of the events the individuals are being reminded to perform.

While this invention is shown in a preferred embodiment, those skilled in the art will recognize that this invention may be practiced in other embodiments as described by the claims herein, and is not restricted to any one embodiment as described herein.

## Claims

1. A method for tracking patient compliance with a regimen, said method comprising the steps of:
maintaining information regarding time and dosage requirements of medication for a patient;
signaling said patient based on said information;
providing a pathway to receive, from said patient, an indication of compliance responsive to said signaling; and
providing to a third party a further indication of an extent to which said patient has complied with said requirements based on one of whether and when said indication has been provided from said patient.

2. The method of claim 1 further comprising the step of creating a secondary database comprising data corresponding to a message and a device address corresponding to said patient, said message and said device address indexed based on a time at which said patient is to be signaled.

3. The method of claim 2 further comprising:
signaling said patient at said time and using said device address with said message in the secondary database.

4. The method of claim 1 further comprising:
providing said patient with a confirmation signal that the patient's indications have been received.

5. The method of claim 1, wherein said indication is one of a plurality of indications expected to be received from said patient further comprising the steps of:
expecting to collect said plurality of indications of compliance from said patient;
generating at least one representation of patient compliance based on ones of said plurality of indications received from said patient; and
providing said patient with at least one confirmation signal to indicate that the ones of the patient's indications have been received.

6. The method of claim 1 further comprising the step of:
providing said further indication across a communications network.

7. The method of claim 1, wherein said indication is one of a plurality of indications expected to be received from said patient and said third party is one of a plurality of third parties further comprising the steps of:
generating a plurality of data values based on collection of ones of said indications from said patient; and
making respective ones of said data values available to respective ones of said plurality of third parties.

8. The method of claim 1 further comprising the steps of:
forming a plurality of messages, each message corresponding to a reminder for said medication at a predetermined time;
pre-storing said plurality of messages prior to transmitting said plurality of messages; and
transmitting each of said plurality of said messages to said patient by retrieving each of said plurality of said messages which have been pre-stored.

9. A method of tracking patient compliance with medication regimens, said method comprising the steps of:
a) maintaining information regarding time and dosage requirements of medications for patients;
b) signaling said patients based on said information;
c) providing respective pathways to receive, from respective patients, respective indications of compliance responsive to respective signaling; and;
d) providing to third parties respective further indications of extents to which said patients have complied with said requirements based on one of whether and when said indications have been provided from said patients.

10. The method of claim 9, further comprising the step of performing statistical analysis based on one of whether and when said indications of compliance have been received from said patients.

11. The method of claim 9, further comprising the step of preventing one of said third parties from receiving information corresponding to one of said further indications of compliance corresponding to one of said patients to which one of said third parties does not correspond.

12. The method of claim 9 further comprising the step of providing respectively different visual interfaces to each of said plurality of third parties.

13. The method of claim 9 further comprising the step of transmitting said indications of compliance and formatting software to each of said plurality of third parties.

14. A storage medium containing a set of instructions to perform a method for tracking patient compliance with a medication regimen, said method comprising:
maintaining information regarding time and dosage requirements of medication for a patient;
signaling said patient based on said information;
providing a pathway to receive from said patient indications of compliance responsive to said signaling; and
providing to a third party a further indication of whether said patient has complied with said requirements based on whether said indication have been received.

15. A computer system for tracking patient compliance with a medication regimen, said system comprising:
means for maintaining information regarding time and dosage requirements of medication for a patient;
means for signaling said patient based on said information;
means for providing a pathway to receive from said patient indications of compliance responsive to said signaling; and
means for providing to a third party a further indication of an extent to which said patient has complied with said requirements based on one of whether and when said indication has been provided from said patient.

16. A method for tracking patient compliance with a medication regimen, said method comprising:
entering time and dosage requirements of medication for a patient into a database;
receiving a signal based on said requirements from a remote location;
transmitting a further signal indicating compliance with the requirements to the remote location; and
giving permission for a third party to receive information regarding compliance with the requirements.

17. A method for tracking patient compliance with a medication regimen for a patent, said method comprising:
requesting information corresponding to said patient compliance from a remote location;
receiving compliance reports indicative of said patient compliance from a remote location; and
viewing compliance data regarding said patient.

18. The method of claim 17 further comprising:
receiving unformatted compliance data and display software from a remote location; and
displaying formatted compliance data using the display software.

19. Apparatus for tracking patient compliance with a medication regimen, said apparatus comprising:
a database for maintaining information regarding time and dosage requirements of medication for a patient;
signaling apparatus for signaling said patient based on said information;
communication apparatus for providing a pathway to receive, from said patient, an indication of compliance responsive to said signaling end;
a transmitter for providing to a third party a further indication of an extent to which said patient has complied with said requirements based on one of whether and when said indication has been provided from said patient.

20. Apparatus for tracking patient compliance according to claim 19, further comprising a secondary database comprising data corresponding to a message and a device address corresponding to said patient, said message and said device address indexed based on a time at which said patient is to be signaled.

21. Apparatus according to claim 20, wherein said patient is signaled at said time and using said device address with said message in the secondary database.

22. Apparatus for tracking patient compliance according to claim 19, further comprising a confirmation unit for providing said patient with a confirmation signal that the patient's indications have been received.

23. The method of claim 1, wherein said patient is signaled and said indication is received from said patient over respectively different kinds of communication medium.

24. A method for tracking compliance of one or more individuals with one or more regimens, said method comprising the steps of:
maintaining information regarding time and event requirements of one or more actions to be taken by said one or more individuals;
signaling said one or more individuals based on said information;
providing one or more pathways to receive, from said one or more individuals respective indications of compliance responsive to said signaling; and
providing to one or more third parties respective further indications of respective extents to which said one more individuals have complied with said requirements based on one of whether and when said respective indications have been provided from said one or more individuals.
